# EUROPEAN PATENT SPECIFICATION

(11) **EP 2 321 648 B1**
(45) Date of publication and mention of the grant of the patent: **05.11.2014**
(21) Application number: 09786657.8
(22) Date of filing: 21.07.2009
(51) Int. Cl.: G01N 33/53

(54) **DIAGNOSIS OF SYSTEMIC DISEASES**
DIAGNOSE SYSTEMISCHER KRANKHEITEN
DIAGNOSTIC DE MALADIES SYSTÉMIQUES

(30) Priority: 21.07.2008 HU 0800448
(43) Date of publication of application: 18.05.2011
(73) Proprietor: University of Pécs, 7622 Pécs, Vasvári Pál u. 4. (HU)
(72) Inventor: NÉMETH, Péter, H-7627 Pécs (HU); CZÖMPÖLY, Tamás, H-7200 Dombóvár (HU); BERKI, Tímea, H-7627 Pécs (HU); CZIRJÁK, László, H-7627 Pécs (HU)
(74) Representative: Svingor, Adam
(86) International application number: PCT/IB2009/053160
(87) International publication number: WO 2010/010514

(56) References cited:
- EP-A1- 0 787 795
- MAUL G G ET AL: "Determination of an epitope of th ediffuse sytemic sclerosis marker antigen DNA topoisomerase I: Sequence similarity with retroviral p30 gag protein suggest a possible cause for autoimmunity in systemic sclerosis" PROCEEDINGS OF THE NATIONAL ACADEMY OF SCIENCES OF USA, NATIONAL ACADEMY OF SCIENCE, WASHINGTON, DC, US, no. 86, 1 November 1989 (1989-11-01), pages 8492-8496, XP002079331 ISSN: 0027-8424
- D'ARPA P ET AL: "Use of molecular cloning methods to map the distribution of epitopes on topoisomerase I (Scl-70) recognized by sera of scleroderma patients" ARTHRITIS AND RHEUMATISM, LIPPINCOTT, PHILADELPHIA, US, [Online] vol. 33, no. 10, 1 January 1990 (1990-01-01), pages 1501-1511, XP009124204 ISSN: 0004-3591 Retrieved from the Internet: URL:http://www3.interscience.wiley.com/cgi -bin/jhome/112142621>
- HU, PAUL Q.: "MOLECULAR RECOGNITION PATTERNS OF SERUM ANTI-DNA TOPOISOMERASE I ANTIBODY IN SYSTEMIC SCLEROSIS" JOURNAL OF IMMUNOLOGY, vol. 173, 2004, pages 2834-2841, XP002550660 cited in the application
- SIMON, D. ET AL: "Naturally occurring and disease-associated autoantibodies against topoisomerase I: a fine epitope mapping study in systemic sclerosis and systemic lupus erythematosus." INTERNATIONAL IMMUNOLOGY, vol. 21, no. 4, 11 February 2009 (2009-02-11), pages 415-422, XP002550661

## Description

The present invention relates to novel diagnostic methods for systemic sclerosis and systemic lupus erythematosus, uses of anti-topoisomerase I and fragments thereof for such diagnosis and diagnostic kits.

### BACKGROUND ART

Systemic sclerosis (SSc) is a systemic autoimmune disorder characterized by immune activation, vascular injury, inflammation, fibrosis of the skin and various internal organs. Activation of the immune system leads to production of disease specific autoantibodies, lymphocyte activation and secretion of various cytokines [1]. The vast majority of SSc patients have antinuclear antibodies, which predominantly recognize DNA topoisomerase I (topo I or TOP I), RNA polymerases, centromere proteins, and U3RNP [2].

Topo I is a 765 amino acid (AA) long DNA-relaxing enzyme which contains five distinct regions: the N-terminal domain (AA 1-215), core subdomains I-II (AA 216-435), core subdomain III (AA 436-636), the linker domain (637-713) and the C-terminal domain (AA 714-765).

Clinically patients with SSc could be classified into two distinct subsets. dcSSc is characterized by extensive fibrosis of the skin, lungs and other internal organs, while in lcSSc vascular abnormalities are dominating and fibrosis is limited [5].

In addition to SSc presence of anti-topo I antibodies has been demonstrated in systemic lupus erythematosus (SLE) patients showing no clinical signs and symptoms of systemic sclerosis [6,7]. These findings suggest that the presence of anti-topo I antibodies could have heterogeneous clinical consequences.

Use of topo-I and topo-I peptides have been proposed in detection of autoantibodies.

In US 5070192 cloning of a topo-I polypeptide is described and detection of autoantibodies in patient samples by binding to said topo-I polypepetide is suggested. In US 5849503 a mutant topo-I polypeptide is described which is thought to be useful for detecting autoantibodies e.g. in an immunoassay.

Later, a number of groups have studied the epitope specificity of anti-topo I antibodies in SSc patients. In these studies, nevertheless, various epitopes in the central and C-terminal part of the molecule [15-20] have been suggested. Reports suggest that an immunodominant region of topo I spans AA 489-573 [15, 16, 19]. However, a study which used recombinant fusion proteins constructed on the basis of the domain structure of topo I demonstrated that the core subdomains I-II is recognized more frequently than core subdomain III [11]. Longitudinal analysis of anti-topo I auto-antibodies revealed that reactivity against these regions is stable [9, 11], though a study using a limited number of sera showed that the regions recognized by anti-topo I auto-antibodies vary over time [10]. Thus several uncertainties existed in the art regarding topo I epitopes in systemic diseases and while epitope specificity of anti-topo I autoantibodies has been studied by a number of groups, there is no report of comparative epitope mapping in patients with dcSSc, lcSSc and SLE and there is no suggestion for a differential diagnosis of these diseases based on autoantibody binding to different epitopes.

### BRIEF DESCRIPTION OF THE INVENTION

The present invention relates to a use of one or more isolated peptide(s) for diagnosis of a systemic autoimmune disorder, said one or more peptide(s) separately or simultaneously comprising at least one of the following epitopes:
a) an epitope having the immunological property of an at least 20 amino acids long segment of the portion of a DNA Topoisomerase I spanning amino acids 5 to 30,
b) an epitope having the immunological property of an at least 20 amino acids long segment of the portion of a DNA Topoisomerase I spanning amino acids 350 to 400,
   said one or more peptide(s) having an amino acid sequence which is at least 70%, 75%, 80%, 85% or 90%, 92%, 94%, 96% or 98% identical with the amino acid sequence of a peptide fragment of a wild type DNA Topoisomerase I,
   wherein
   - if binding of autoantibodies of a sample from a patient to epitope according to a) is detected, it is considered as indicative of diffuse cutane systemic scleroderma (dsSSC), preferably a late onset thereof, in said patient,
   - if binding of autoantibodies of a sample from a patient to epitope according to b) is detected, it is considered as indicative of systemic lupus erithematosus (SLE), preferably an SLE with Raynaud's phenomenon, in said patient.

In preferred embodiments, one or more isolated peptide(s) also comprise
c) an epitope having the immunological property of the at least 20 amino acids long segment of the portion of said DNA Topoisomerase I spanning amino acids 451 to 593, and wherein
   - if binding of autoantibodies of a sample from a patient to epitope according to a) and c) is detected, and preferably binding of autoantibodies of said patient sample to epitope according to b) is not detected, it is considered as indicative of diffuse cutane systemic scleroderma (dsSSC) in said patient or preferably a late onset thereof,
   - if binding of autoantibodies of a sample from a patient to epitope according to b) and c) is detected, and preferably binding of autoantibodies of said patient sample to epitope according to a) is not detected, it is considered as indicative of systemic lupus erithematosus (SLE) in said patient or preferably an SLE with Raynaud's phenomenon.

Preferably, the epitope of said peptide is different in not more than 10, 9, 8, 7, 6, 5, 4, 3, 2 or 1 amino acids from the respective epitope of the wild type sequence.

In a further preferred embodiment
- epitope according to a) is an at least 20 amino acids long segment of the portion of said DNA Topoisomerase I spanning amino acids 5 to 30, and/or
- epitope according to b) is an at least 20 amino acids long segment of the portion of said DNA Topoisomerase I spanning amino acids 350 to 400, and/or, preferably
- epitope according to c) is an at least 20 amino acids long segment of the portion of said DNA Topoisomerase I spanning amino acids 451 to 593.

In a preferred embodiment all the epitopes are present on separate peptides.

In an embodiment peptides epitopes a) and b) are present on different peptides. In an embodiment peptides may be used wherein epitopes according to a) and c) are present on the same peptide and epitope according to b) is present on a different peptide, or epitopes according to b) and c) are present on the same peptide and epitope according to a) is present on a different peptide.

If binding of an antibody to an epitope can be detected specifically, in an alternative embodiment, epitopes according to a) and c) may be present on one peptide and epitopes according to b) and c) may be present on another peptide.

If desired, appropriate antibody standards are to be used.

In a further aspect the invention relates to a kit, preferably a diagnostic kit, for use in the diagnosis of a systemic autoimmune disorder, said kit comprising one or more peptides as defined in any of the previous claims,
said one or more peptide(s) separately or simultaneously comprising at least two of the following epitopes:
a) an epitope having the immunological property of an at least 20 amino acids long segment of the portion of a DNA Topoisomerase I spanning amino acids 5 to 30,
b) an epitope having the immunological property of an at least 20 amino acids long segment of the portion of a DNA Topoisomerase I spanning amino acids 350 to 400, and preferably
c) an epitope having the immunological property of an at least 20 amino acids long segment of the portion of a DNA Topoisomerase I spanning amino acids 451 to 593.

A preferred kit of the invention comprises
- at least one peptide selected from
   a) a peptide comprising an epitope having the immunological property of an at least 20 amino acids long segment of the portion of a DNA Topoisomerase I spanning amino acids 5 to 30,
   b) a peptide comprising an epitope having the immunological property of an at least 20 amino acids long segment of the portion of a DNA Topoisomerase I spanning amino acids 350 to 400, and
- a reference peptide comprising an epitope having the immunological property of an at least 20 amino acids long segment of the portion of a DNA Topoisomerase I spanning amino acids 451 to 593; and optionally
- means for detecting binding of patient autoantibodies to a peptide epitope.

Preferably,
- the peptide according to a) is a peptide fragment of said DNA Topoisomerase I spanning amino acids 5 to 30,
- the peptide according to b) is a peptide fragment of said DNA Topoisomerase I spanning amino acids 350 to 400, and
- the peptide according to c) is a peptide fragment of said DNA Topoisomerase I spanning amino acids 451 to 593.

In a further aspect the invention relates to a diagnostic method useful for differential diagnosis of systemic autoimmune disorders comprising the steps of
i) contacting a sample obtained from a patient with one or more peptide(s) comprising at least one of the following epitopes:
   a) an epitope having the immunological property of an at least 20 amino acids long segment of the portion of said DNA Topoisomerase I spanning amino acids 5 to 30,
   b) an epitope having the immunological property of an at least 20 amino acids long segment of the portion of said DNA Topoisomerase I spanning amino acids 350 to 400
   said one or more peptide(s) having an amino acid sequence which is at least 70%, 75%, 80%, 85% or 90%, 92%, 94%, 96% or 98% identical with the amino acid sequence of a peptide fragment of a wild type DNA Topoisomerase I,
   wherein
   - if binding of autoantibodies of said sample to epitope according to a) is detected, it is considered as indicative of diffuse cutane systemic scleroderma (dsSSC), preferably a late onset thereof, in said patient,
   - if binding of autoantibodies of said sample to epitope according to b) is detected, it is considered as indicative of systemic lupus erithematosus (SLE), preferably an SLE with Raynaud's phenomenon, in said patient.

In a preferred method of the invention, the one or more isolated peptide(s) also comprise
c) an epitope having the immunological property of the at least 20 amino acids long segment of the portion of said DNA Topoisomerase I spanning amino acids 451 to 593, and wherein
   - if binding of autoantibodies to epitope according to a) and c) is detected, and preferably binding of autoantibodies of said patient sample to epitope according to b) is not detected, it is considered as indicative of diffuse cutane systemic scleroderma (dsSSC) in said patient or preferably a late onset thereof,
   - if binding of autoantibodies to epitope according to b) and c) is detected, and preferably binding of autoantibodies of said patient sample to epitope according to a) is not detected, it is considered as indicative of systemic lupus erithematosus (SLE) in said patient or preferably an SLE with Raynaud's phenomenon.

Preferably the one or more peptide(s) as defined above are used in the methods, uses and or kits of the invention.

In a preferred embodiment the sample is a blood sample, preferably a serum sample.

### DEFINITIONS

*"An epitope having the immunological property of a segment of a portion of DNA Topoisomerase I"* is understood herein as an epitope capable of binding an antibody also capable of binding to said segment, preferably a patient antibody, more preferably a patient antibody of a patient having a systemic disease, preferably a systemic sclerosis or systemic lupus erythematosus.
*"DNA Topoisomernse I"* in accordance with the present invention can be a wild type DNA Topoisomerase I (EC 5.99.1.2.) from eukaryotic, preferably vertebrate, preferably mammalian, more preferably human source, or having the sequence identical with such a wild type DNA Topoisomerase I, or a mutant having a sequence identity of at least 50%, 60%, 70%, 75%, 80%, 85%, 90%, 93%, 95%, 97%, 98%, 99% or 100% therewith. Preferably at least one immunological property of said mutant DNA Topoisomerase I is maintained or one or more antibody capable of binding to the wild type protein is capable of binding to the mutant protein as well. Said DNA Topoisomerase I can be isolated from a natural source or recombinantly prepared.
   The term *"peptide",* as applied herein denotes any sequence of amino acids having an epitope for autoantibodies to eukaryotic, preferably vertebrate, preferably mammalian, more preferably human topoisomerase I (topo I). Preferably, the sequence of amino acids of said peptide is encoded by all or part of a polynucleotide, e.g. a cDNA, encoding said topo I or the amino acids sequence of said peptide having a sequence identity of at least 50%, 60%, 70%, 75%, 80%, 85%, 90%, 93%, 95%, 97%, 98%, 99% or 100% therewith.
*"Autoantihodies"* are antibodies of a subject raised against or capable of binding to a protein of said subject.
*"Patient",* as used herein, refers to an animal or human subject who is treated, diagnosed or a body sample of whom is analyzed, or to be treated, diagnosed or analyzed.

### BRIEF DESCRIPTION OF THE FIGURES

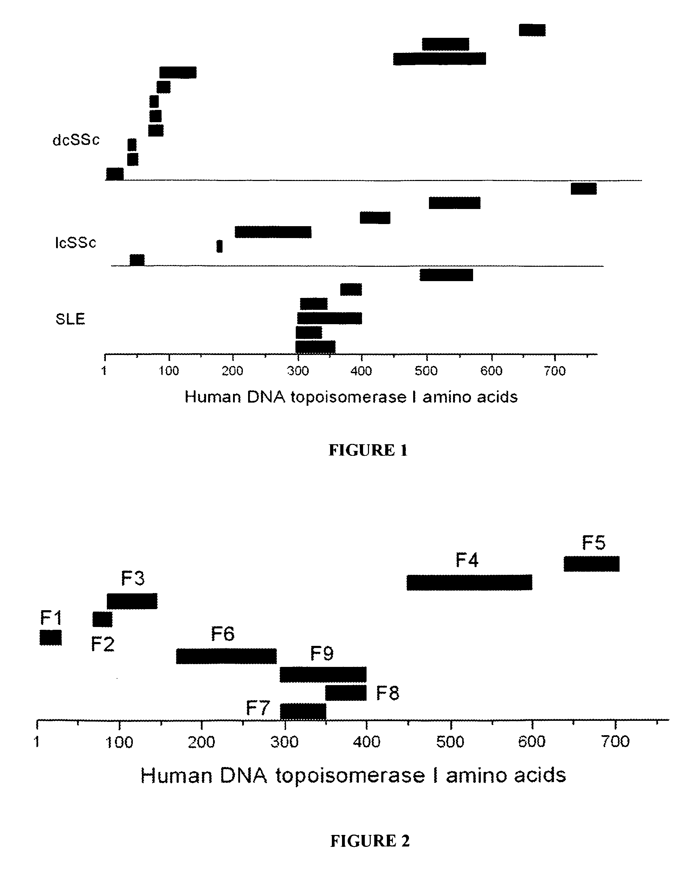

**Figure 1** The pattern of recognized topoisomerase I (topo I) epitopes is different between dcSSc, lcSSc and SLE patients. Deduced amino acid sequences of phage clones selected with IgG purified from 5 diffuse cutaneous systemic sclerosis (dcSSc), 6 limited cutaneous systemic sclerosis (IcSSc) and 4 systemic lupus erythematosus (SLE) patients are plotted along the human topo I sequence.
**Figure 2** Recombinant topoisomerase I-maltose binding protein fusion constructs used in this study.

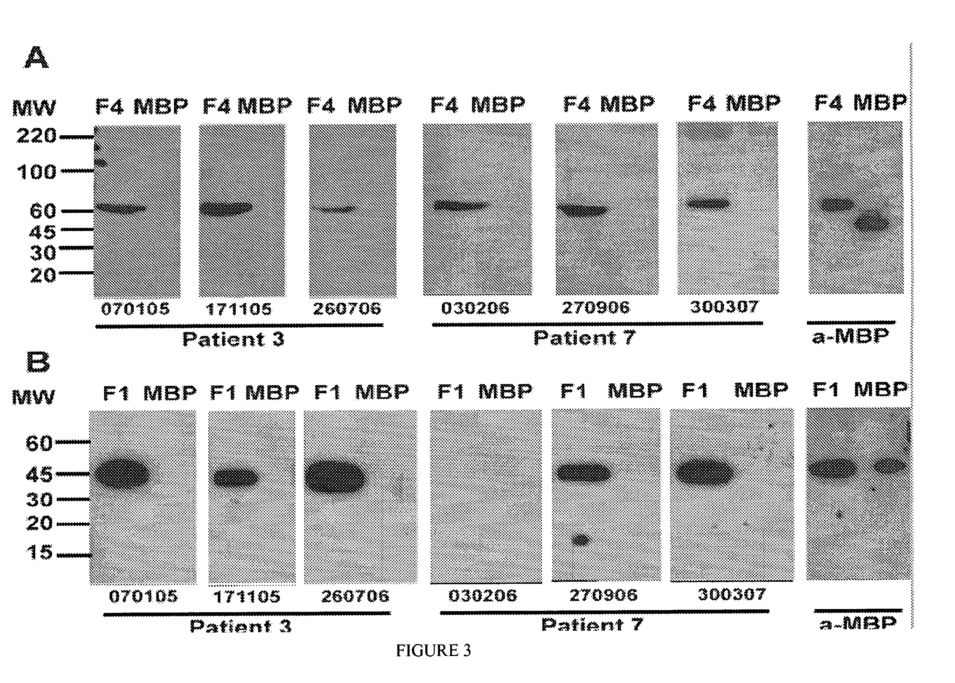

**Figure 3** Immunoblots using topoisomerase I (topo I) fusion proteins F4 and F1 as antigens. Purified recombinant fusion proteins (panel A: F4, panel B: F1) or maltose binding protein (MBP) were separated on a 10% SDS-polyacrylamide gel and transferred to nitrocellulose membranes. The membranes were cut into strips and probed with serial serum samples (obtained at dates indicated) of patients or with an anti-MBP antibody (a-MBP). MW: molecular weight marker (kDa).

### DETAILED DESCRIPTION OF THE INVENTION

The role of anti-topo I antibodies in pathogenesis of SSc is not fully understood, however, it can be assumed based on the present invention that immune response against topo I may differ among anti-topo I positive patients leading to production of anti-topo I autoantibodies with different epitope specificity.

In the prior art various recombinant topo I fragments were used, which were designed either on the basis of topo I domain structure or antigenicity prediction, both of which could miss possible epitopes [12-18]. For example, Hu et al. [21], who used a fusion protein covering the entire length (AA 1-213) of the N-terminal domain, showed that this part of the molecule is not targeted by anti-topo I antibodies. To the contrary, other previous studies performed with fusion proteins covering the N-terminal domain starting from AA 70 reported that this part of the molecule is recognized by anti-topo I antibodies [13, 16, 20].

These seemingly contradictory results may be due to the different methods and antigen constructs applied, and quite probably to possible conformational factors which could influence the accessibility of short epitopes buried in the tertiary structure. It is noted here in advance that the majority of new epitope-containing fragments, including fragment F1, identified by the present inventors at the N-terminal part spans only 20-30 AA.

Thus, the present inventors have chosen a different strategy and have constructed an antigen fragment library of topo I displayed on bacteriophage lambda and screened this library with sera of dcSSc, lcSSc and SLE patients. Regions of topo I selected from the library were expressed as recombinant fusion proteins and were further tested with patients' sera. Longitudinal analysis of epitope specificities has been performed and compared with clinical findings.

On the basis of fragments selected from the phage displayed antigen fragment library we expressed nine topo I-MBP fusion proteins and tested these fragments with 67 anti-topo I antibody positive patients' sera. According to our results recognition of the majority of fragments (F2, F3, F5-7, F9) is characteristic for the individual patient sera used for library screening, instead of being characteristic for the given disease subgroup. This is in agreement with result of Henry et al, who found both individual and longitudinal differences in the recognized topo I epitopes [20]. However, besides fragment F4 (AA 451-593) detected in all patient sera tested, fragment F1 (AA 5-30) was specifically recognized by a subset of dcSSc patients' sera, and fragment F8 (350-400) was recognized by SLE patients, indicating that these fragments could represent characteristic epitopes for dcSSc and SLE, respectively.

Thus, data obtained with a phage display based approach clearly demonstrate that the pattern of recognized epitopes is different between dcSSc, lcSSc and SLE patients. The finding of a common fragment recognized by all Topo I reactive patients' was located in the region of AA 451-593 (fragment F4), which is in agreement with previously published results [12,13]. However, according to the present results immunological assays, e.g. ELISA performed with fragment F4 may represent a more sensitive tool to detect anti-topo I autoantibodies than conventional ELISA systems using full length antigen.

In addition to an immunodominant part of topo I (fragment F4), two new regions have been identified which were previously not shown to be targeted by anti-topo I antibodies. dcSSc patients recognized several short fragments (spanning AA 5-145) at the N-terminal part of the molecule. Specifically, fragment F1 (AA 5-30) was recognized by a subset of dcSSc patients' sera, while fragment F8 was recognized by SLE patients.

Moreover, analysis of clinical data suggests that autoantibodies against fragment F1 may indicate the evolution of the disease in late stage dcSSc. This fragment F1 contains an experimentally proven granzyme B cleavage site [22]. Thus it is possible that in vivo cleavage of topo I by granzyme B released during T cell mediated cytotoxic responses results in the formation of a neo-antigenic determinant represented by fragment F1. In vitro assays using the full length antigen or the full length N-terminal domain may fail to detect antibodies recognizing these short epitopes.

Analysis of clinical data failed to demonstrate clear associations between anti-topo I antibody epitope specificity and clinical presentation of the disease. However, without being bound by theory, the difference in the duration of disease between anti-F1 antibody positive and negative dcSSc patients, together with findings of our longitudinal analysis, may indicate that the anti-topo I immune response is initiated against the immunodominant part of the molecule (fragment F4), and may target the N-terminal part later during the course of the disease. Thus autoantibodies against fragment F1 may represent a new marker of late stage dcSSc. The mechanism of this "epitope spreading" and factors which facilitate this in dcSSc remain to be further investigated.

Moreover, comparison of clinical data of the 4 F8 positive and the 261 F8 negative SLE patients suggested that SLE patients with antibody against fragment F8 have Raynaud's phenomenon and a milder presentation of the disease (lack of arthritis, central nervous system and kidney involvement). In a preferred embodiment in the diagnostic method or use or in the kits of the invention the epitopes recognized by the autoantibodies are present on separate peptides. In a further preferred embodiment antibody standards may be prepared by usual immunological methods. It is well within the skills of a person skilled in the art to detect autoantibody binding. It is immediately apparent for a skilled person that both kinetic methods as well as method characterizing binding affinity or avidity are applicable. For example, any of the methods below, without any limitation, are applicable:
an immunoassay, e.g. ELISA, RIA, lateral flow, immunoprecipitation,
a binding assay, e.g. Biacore, fluorescence quenching,
a spectrophotometric method, e.g. FT-IR, circular dichroism, NMR,
a physico-chemical method, e.g. calorimetry, ultracentrifugation etc.

In a preferred embodiment of the diagnostic method is carried out in the form of an immunoassay, like RIA or DELPHIA or preferably in an immunosorbent assay, like ELISA.

A detailed teaching can be found e.g. in the book Autoantibodies, by Yehuda Shoenfeld, Pier-Luigi Meroni, M. Eric Gershwin, Elsevier, second edition.

In certain embodiments the object may be to provide an assay which selectively differentiates among patient autoantibodies which a related to the disorder and other antibodies. Setting the sensitivity of the assay is well within the skills of a person skilled in the art, who will be able to find appropriate control peptides. Examples for such peptides are provided herein. A positive inner control is in fact F4 fragment itself. As a negative control other, non-immunogenic fragments or other proteins, like MBP can be used.

In a preferred variant IgM or IgG are measured as autoantibodies, provided that the patients are not deficient in any of these types.

The invention also relates to kits for performing the diagnostic methods as outlined above. These kits may comprise the parts as mentioned above or as useful in carrying out the methods outlined above. A kit necessarily comprises peptides carrying the epitope and at least instructions for use.

The invention is described below more specifically by non-limiting examples. The skilled person will understand, however, that other embodiments based on the idea and within the scope of the invention can be reduced in the practice based on knowledge in the art and his/her general knowledge. Moreover, the teaching of the cited references is specifically incorporated herein.

### EXAMPLES

### METHODS

### Patients and controls

From the 293 patients in our total scleroderma cohort, 59 SSc cases were selected which showed anti-topoisomerase antibody positivity on a conventional ELISA test (Hycor, Vienna, Austria) (34 of these patients were classified as having dcSSc and 25 were diagnosed IcSSc). Out of 265 SLE patients tested for the presence of anti-topo I antibodies with a conventional ELISA kit 8 showed positive reaction and were selected for the present study. 177 serum samples from 59 SSc patients (3 from each patient) and 24 serum samples from 8 SLE patients were obtained between 2004 and 2007 at 6 to 12 months intervals. The patients' clinical data were encoded using our standard protocol [8].

For controls 146 anti-topo I antibody negative serum samples from healthy women and men of various ages were used. Furthermore 110 age matched patients with different inflammatory rheumatic diseases (8 vasculitis, 40 seronegative spondylarthritis, 11 myositis, 11 Sjögren syndrome, 10 psoriatic arthritis, 20 rheumatoid arthritis, 10 polymyalgia rheumatica) were also investigated.

The study has been approved by the Ethical Committee of the Medical Center of the University of Pécs. Informed consent has been obtained from all patients and healthy individuals.

### Construction and affinity selection of topo I antigen fragment library

The coding region of full length human topo I was amplified by PCR from cDNA reverse transcribed from total RNA. The PCR product was cloned into a T/A vector using the InsT/Aclone PCR Product Cloning Kit (Fermentas, Vilnius, Lithuania). Library construction was done using the lambdaD-bio phage display vector [9] with minor modifications as described previously [10]. The primary topo I library contained 2x10⁷ insert bearing independent clones; titer of the amplified library was 3x10¹¹/ml. Affinity selection of topo I antigen fragment library with 5 dcSSc, 6 lcSSc and 4 SLE patient derived IgG purified on protein G sepharose (Amersham Pharmacia, Uppsala, Sweden) was performed essentially as described [11]. After the third round of selection individual clones were picked up for further propagation and DNA sequencing.

### Expression of recombinant topo I fusion proteins

Selected fragments of topo I were expressed as recombinant maltose binding protein (MBP) fusion proteins using the pMAL Protein Fusion and Purification system (New England Biolabs, Ipswich, UK). cDNAs coding for AA 5-30 (F1), 69-92 (F2), 87-145 (F3), 450-600 (F4), 640-705 (F5), 170-290 (F6), 295-350 (F7), 350-400 (F8), 295-400 (F9) were amplified with PCR primers containing EcoRI and BamHI restriction sites and cloned into the pMal-c2 vector. Fusion proteins were purified from bacterial lysates with affinity chromatography on amylose resin according to the manufacturer's instruction (New England Biolabs, Ipswich, UK), and integrity of purified proteins was verified by SDS-PAGE on a 10% gel followed by Coomassie brilliant blue staining.

### Elisa

96-well polystyrene plates (Nunc, Roskilde, Denmark) were coated with recombinant topo I fragments or with MBP in PBS at a concentration of 10 µg/ml. Plates were washed with wash buffer (PBS, 0.05% Tween-20) and blocked with 3% non-fat dry milk in wash buffer for 1 h. Serum samples were incubated in triplicates at 1:250 dilutions in wash buffer containing 2% non-fat dry milk for 1 h. Finally, the plate was incubated with HRP conjugated anti-human-IgG secondary antibody (Dako, Glostrup, Denmark) for 60 min. The reaction was developed with o-phenylenediamine (Sigma-Aldrich, Budapest, Hungary), and optical density (OD) was measured at 492 nm. In order to determine cut off values for further measurements sera of 146 healthy controls (previously tested negative for anti-topo I antibody with a commercial ELISA kit) were tested on all topo I fragments and MBP. Reactivity of healthy controls' sera with topo I fragments and MBP was shown to be minimal (OD₄₉₂ 0.019-0.032), and a cut off value of 0.1 have been chosen for further measurements.

### Immunoblots

Purified MBP fusion proteins or MBP (40 µg/ml) diluted 1:1 with SDS sample buffer were boiled for 10 minutes, separated on a 10% SDS-polyacrylamide gel and transferred to nitrocellulose membranes. After blocking with 5% non-fat dry milk (Bio-Rad, Budapest, Hungary) in wash buffer (100mM NaCl, 10mM Tris-base pH 7.4, 0.1% Tween 20) for 1 h, membranes were incubated for 1 h with sera diluted 1:500 in 2% non-fat dry milk in wash buffer. After washing, HRP-conjugated anti-human-IgG diluted at 1:2000 was added for 1 hour. For detection of MBP fusion proteins membrane strips were first incubated with rabbit anti-MBP antibody (New England Biolabs, Ipswich, UK) (1:5000), followed by incubation with HRP-conjugated goat anti-rabbit antibody (1:2000). Membranes were developed with SuperSignal West Pico Chemiluminescent (Pierce, Rockford, USA) substrate and exposed to x-ray films.

### Statistical analysis

Categorical data were analyzed by the Chi-square test. To investigate the possible differences between patient groups, frequency and mean values of continuous variables were tested by Student's t test. Spearman's rank correlation coefficient was used to examine the relationship between the values of optical density and continuous variables. A p value less than 0.05 was considered statistically significant. Statistical analyses were conducted using SPSS statistical software package.

Thus, while the present diagnostic method may not be applicable in each case, if the patient has topo-I reactive antibodies, it can safely differentiate between at least dcSSc and SLE.

### RESULTS

### Epitope mapping of anti-topo I antibodies with phage displayed topo I library

For identification of epitopes recognized by anti-topo I antibodies we have constructed a topo I antigen fragment library displayed on bacteriophage lambda, and subsequently screened this library with individual IgGs purified from sera of anti-topo I positive patients (5 dcSSc, 6 IcSSc and 4 SLE patient). After the third round of affinity selection inserts of 60 clones (30 from each patient group) were sequenced. Alignment of deduced amino acid sequences with human topo I showed that the pattern of recognized epitopes is different between dcSSc, IcSSc and SLE patients (fig 1).

### Recognition of recombinant topo I fusion proteins by anti-topo I antibodies

In order to verify results obtained by affinity selection of the topo I antigen fragment library we have constructed recombinant topo I-MBP fusion proteins. On the basis of fragments identified by library selection nine fusion proteins have been constructed and expressed (fig 2). Recognition of these fusion proteins was tested with sera of 67 anti-topo I antibody positive patients (34 dcSSc, 25 lcSSc and 8 SLE) including those which have been used for library selection by ELISA. The results are summarized in table 1.

**Table I Recognition frequencies of recombinant topoisomerase I (topo I) fragments determined by ELISA using anti-topo I antibody positive patients' sera**

| Topo I fragments (amino acids) | dcSSc (n = 34) | lcSSc (n = 25) | SLE (n = 8) |
|---|---|---|---|
| F1 (5-30) | 9 (26%) | 1 (4%) | 0 |
| F2 (69-92) | 1 (3%) | 1 (4%) | 0 |
| F3 (87-145) | 1 (3%) | 0 | 0 |
| F4 (450-600) | 34 (100%) | 25 (100%) | 8 (100%) |
| F5 (640-705) | 2 (6%) | 0 | 0 |
| F6 (170-290) | 2 (6%) | 2 (8%) | 0 |
| F7 (295-400) | 0 | 0 | 1 (12%) |
| F8 (350-400) | 0 | 0 | 4 (50%) |
| F9 (295-350) | 0 | 0 | 1 (12%) |

Numbers indicate individual patients positive for the given fragments, numbers in parentheses indicate percentage of positive sera (dcSSc: diffuse cutaneous systemic sclerosis; lcSSc: limited cutaneous systemic sclerosis; SLE: systemic lupus erythematosus).

Fragment F4 (AA 450-600) was recognized by all of the 67 patients' sera. Fragment F1 (AA 5-30) was recognized by 9 of 34, 1 of 25 and 0 of 8 dcSSc, IcSSc and SLE patients, respectively. Fragment F8 (AA 350-400) was recognized by 4 of 8 SLE patients and none of the SSc patients.

### Longitudinal analysis of topo I epitope reactivity

To determine whether antibody responses against fragments F1, F4 and F8 remain constant over time, we measured antibody reactivity against these fragments by ELISA in 3 serial serum samples of each patient. Results of ELISA were confirmed by western blot in 10 F1 positive patients, 10 randomly chosen F4 positive patients and 4 F8 positive patients. Each serum sample was also examined by conventional anti-Scl-70 ELISA.

Longitudinal analysis showed that reactivity to fragment F4 was stable in 61 cases (94%). 18 out of the 67 patients had at least one serum sample with no antibody response against topo I measured by the conventional anti-Scl-70 ELISA. Results of ELISA with fragment F4 were confirmed by western blot, which showed that each sera positive for F4 reactivity in ELISA were also positive in western blot (fig 3A).

The reactivity to F1 fragment varied over time. In 4 cases the earliest serum samples did not have any detectable antibodies against F1, but the immunoreaction became positive and stronger over time. In one case reactivity against F1 appeared in the second sample but was absent in the following one. All serum samples of the remaining 4 dcSSc patients were positive for anti-F1 antibody. Results of ELISA were confirmed by western blot, which showed a perfect correlation of results obtained by the two methods (fig 3B).

Among the 4 SLE patients positive for antibody against F8 the reactivity was stable in one patient, and changed in the remaining three patients.

### Clinical findings

Statistical analyses of clinical data (extent of skin involvement, hand contractures, azotemia and/or malignant hypertension, cardiac involvement, dysmotility and stricture/dilatation of esophagus, extent of lung fibrosis, forced vital capacity) showed no association between anti-topo I antibody epitope specificity and clinical presentation of SSc. However, there was a significant difference between the F1 negative and F1 positive groups of SSc patients in average age (F1 negative (number of patients: 49): 54.8 ± 13.5 years; F1 positive (number of patients: 10): 63.9 ± 9.4 years; p=0.048) and the duration of the disease (F1 negative (number of patients: 49): 10.0 ± 7.3 years; F1 positive (number of patients: 10): 17.1±12.9 years; p=0.019). Moreover, average age of dcSSc patients possessing sera positive for F1 fragment was significantly higher compared to the patients showing no detectable levels of antibody against F1 (F1 negative (number of patients: 25): 51.9 ± 14.3 years; F1 positive (number of patients: 9): 63.7 ± 10.0 years; p=0.03).

To investigate whether antibodies against peptide F1 were specific for SSc patients and the appearance of them is not merely a consequence of aging, sera from 64 age matched (average age: 62.4 ± 5.4 years) healthy controls were tested for antibodies against F1. Only one serum sample was found positive. To test whether the presence of antibodies against fragment F1 is specific for SSc, sera from 110 age matched (average age: 65.5 ± 4.8 years) patients with different connective tissue diseases were also tested and only 4 serum samples (2 seronegative spondylarthritis, 1 myositis, 1 Sjögren syndrome) were found positive.

Comparison of clinical data of the 4 F8 positive and the 261 F8 negative SLE patients suggested that SLE patients with antibody against fragment F8 have Raynaud's phenomenon and a milder presentation of the disease (lack of arthritis, central nervous system and kidney involvement).

In summary, it has been demonstrated that in addition to an immunodominant part of topo I, sera of patients with dcSSc, lcSSc and SLE recognize distinct topo I epitopes. We have shown that recognition of the majority of fragments is characteristic for the individual patient, instead of being characteristic for the given disease subgroup. However, fragment F1 (AA 5-30) was specifically recognized by a subset of dcSSc patients' sera, and fragment F8 (350-400) was recognized by SLE patients, indicating that fragment F1 and F8 could represent characteristic epitopes for dcSSc and SLE, respectively.
**Funding:** National Health Fund (ETT: 32/KO/2004)

### REFERENCES

1. Varga J, Abraham D. Systemic sclerosis: a prototypic multisystem fibrotic disorder. J Clin Invest 2007;117:557-67.
2. Okano Y. Antinuclear antibody in systemic sclerosis (scleroderma). Rheum Dis Clin North Am 1996;22:709-35.
3. Steen VD, Powell DL, Medsger TA Jr. Clinical correlations and prognosis based on serum autoantibodies in patients with systemic sclerosis. Arthritis Rheum 1988;31:196-203.
4. Kuwana M, Kaburaki J, Okano Y, Tojo T, Homma M. Clinical and prognostic associations based on serum antinuclear antibodies in Japanese patients with systemic sclerosis. Arthritis Rheum 1994;37:75-83.
5. LeRoy EC, Black C, Fleischmajer R, Jablonska S, Krieg T, Medsger TA Jr, et al. Scleroderma (systemic sclerosis): classification, subsets and pathogenesis. J Rheumatol 1988;15:9202-5.
6. Gussin HA, Ignat GP, Varga J, Teodorescu M. Anti-topoisomerase I (anti-Scl-70) antibodies in patients with systemic lupus erythematosus. Arthritis Rheum 2001;44:376-83.
7. Hamidou MA, Audrain MA, Masseau A, Agard C, Moreau A. Anti-topoisomerase I antibodies in systemic lupus erythematosus as a marker of severe nephritis. Clin Rheunantol 2006;25:542-3.
8. Czirják L, Kumánovics G, Varjú C, Nagy Z, Pákozdi A, Szekanecz Z et al. Survival and causes of death in 366 Hungarian patients with systemic sclerosis. Ann Rheum Dis. 2008;67:59-63.
9. Ansuini H, Cicchini C, Nicosia A, Tripodi M, Cortese R, Luzzago A. Biotin-tagged cDNA expression libraries displayed on lambda phage: a new tool for the selection of natural protein ligands. Nucleic Acids Res 2002;30: e78.
10. Czömpöly T, Olasz K, Simon D, Nyárády Z, Pálinkás L, Czirják L et al. A possible new bridge between innate and adaptive immunity: Are the anti-mitochondrial citrate synthase autoantibodies components of the natural antibody network? Mol Immunol 2006;43:1761-8.
11. Santini C, Brennan D, Mennuni C, Hoess RH, Nicosia A, Cortese R et al. Efficient display of an HCV cDNA expression library as C-terminal fusion to the capsid protein D of bacteriophage lambda. J Mol Biol 1998;282:125-35.
12. D'Arpa, P, White-Cooper H, Cleveland DW, Rothfield NF, Earnshaw WC. Use of molecular cloning methods to map the distribution of epitopes on topoisomerase I (Scl-70) recognized by sera of scleroderma patients. Arthritis Rheum 1990;33:1501-11.
13. Verheijen, R, Van den Hoogen F, Beijer R, Richter A, Penner E, Habets WJ et al. A recombinant topoisomerase I used for autoantibody detection in sera from patients with systemic sclerosis. Clin Exp Immunol 1990;80:38-43.
14. Piccinini G, Cardellini E, Reimer G, Arnett FC, Durban E. An antigenic region of topoisomerase I in DNA polymerase chain reaction-generated fragments recognized by autoantibodies of scleroderma patients. Mol Immunol 1991;28:333-9.
15. Cram DS, Fisicaro N, McNeilage LJ, Coppel RL, Harrison LC. Antibody specificities of Thai and Australian scleroderma sera with topoisomerase I recombinant fusion proteins. J Immunol 1993;151:6872-81.
16. Kuwana M, Kaburaki J, Mimori T, Tojo T, Homma M. 1993. Autoantigenic epitopes on DNA topoisomerase I: clinical and immunogenetic associations in systemic sclerosis. Arthritics Rheum 1993;36:1406-13.
17. Seelig HP, Schroter H, Ehrfeld H, Renz M. Autoantibodies against topoisomerase I detected with the natural enzyme and overlapping recombinant peptides. J Immunol Methods 1993;165:241-52.
18. Kato T, Yamamoto K, Takeuchi H, Okubo M, Hara E, Nakada S, et al. Identification of a universal B cell epitope on DNA topoisomerase I, an autoantigen associated with scleroderma. Arthritis Rheum 1993;36:1580-7.
19. Kuwana M, Kaburaki J, Medsger TA Jr, Wright TM. An immunodominant epitope on DNA topoisomerase I is conformational in nature: heterogeneity in its recognition by systemic sclerosis sera. Arthritis Rheum 1999;42:1179-88.
20. Henry PA, Atamas SP, Yurovsky VV, Luzina I, Wigley FM, White B. Diversity and plasticity of the anti-DNA topoisomerase I autoantibody response in scleroderma. Arthritis Rheum 2000;43:2733-42.
21. Hu PQ, Fertig N, Medsger TA Jr, Wright TM. Molecular recognition patterns of serum anti-DNA topoisomerase I antibody in systemic sclerosis. J Immunol 2004;173:2834-41.
22. Casciola-Rosen L, Andrade F, Ulanet D, Wong WB, Rosen A. Cleavage by granzyme B is strongly predictive of autoantigen status: implications for initiation of autoimmunity. J Exp Med 1999;190:815-26.
23. Redinbo, M. R., Stewart, L., Kuhn, P., Champoux, J. J. and Hol, W. G. J. 1998. Crystal structures of human topoisomerase I in covalent and noncovalent complexes with DNA. Science 279:1504.

## Claims

1. Use of one or more isolated peptide(s) for diagnosis of a systemic autoimmune disorder, said one or more peptide(s) being selected from:
a) a peptide comprising an epitope capable of binding a patient antibody of a patient having diffuse cutane systemic scleroderma (dsSSC), said epitope being identical with or different in not more than 4 amino acids from an at least 20 amino acids long segment of the portion of a DNA Topoisomerase I spanning amino acids 5 to 30,
b) a peptide comprising an epitope capable of binding a patient antibody of a patient having systemic lupus erithematosus (SLE), said epitope being identical with or different in not more than 4 amino acids from an at least 20 amino acids long segment of the portion of a DNA Topoisomerase I spanning amino acids 350 to 400,
wherein the epitopes are present on separate peptides,
said one or more peptide(s) having an amino acid sequence which is at least 80% or at least 90% identical with the amino acid sequence of a peptide fragment of a wild type DNA Topoisomerase I, wherein
- if binding of autoantibodies of a sample from a patient to epitope according to a) is detected, it is considered as indicative of diffuse cutane systemic scleroderma (dsSSC), preferably a late onset thereof, in said patient,
- if binding of autoantibodies of a sample from a patient to epitope according to b) is detected, it is considered as indicative of systemic lupus erithematosus (SLE), preferably an SLE with Raynaud's phenomenon, in said patient.

2. The use according to claim 1 wherein the one or more isolated peptide(s) also comprise
c) a peptide comprising an epitope capable of binding a patient antibody of a patient having systemic sclerosis (SSC) or systemic lupus erythematosus (SLE), said epitope being identical with or different in not more than 4 amino acids from the at least 20 amino acids long segment of the portion of said DNA Topoisomerase I spanning amino acids 451 to 593,
said peptide having an amino acid sequence which is at least 80% or at least 90% identical with the amino acid sequence of a peptide fragment of a wild type DNA Topoisomerase I,
and wherein
- if binding of autoantibodies of a sample from a patient to epitope according to a) and c) is detected, and preferably binding of autoantibodies of said patient sample to epitope according to b) is not detected, it is considered as indicative of diffuse cutane systemic scleroderma (dsSSC) in said patient or preferably a late onset thereof,
- if binding of autoantibodies of a sample from a patient to epitope according to b) and c) is detected, and preferably binding of autoantibodies of said patient sample to epitope according to a) is not detected, it is considered as indicative of systemic lupus erithematosus (SLE) in said patient or preferably an SLE with Raynaud's phenomenon.

3. The use of claim 1 or 2 wherein the epitope of any of said peptide is different in not more than 2 or 1 amino acids from the respective epitope of the wild type sequence.

4. The use of any of claims 1 to 3, wherein
- epitope according to a) is an at least 20 amino acids long segment of the portion of said DNA Topoisomerase I spanning amino acids 5 to 30,
- epitope according to b) is an at least 20 amino acids long segment of the portion of said DNA Topoisomerase I spanning amino acids 350 to 400, and preferably
- epitope according to c) is an at least 20 amino acids long segment of the portion of said DNA Topoisomerase I spanning amino acids 451 to 593.

5. A diagnostic kit for use in the diagnosis of a systemic autoimmune disorder, said kit comprising one or more isolated peptides as defined in any of the previous claims,
said one or more isolated peptide(s) being selected from:
a) a peptide comprising an epitope capable of binding a patient antibody of a patient having diffuse cutane systemic scleroderma (dsSSC), said epitope being identical with or different in not more than 4 amino acids from an at least 20 amino acids long segment of the portion of a DNA Topoisomerase I spanning amino acids 5 to 30,
b) a peptide comprising an epitope capable of binding a patient antibody of a patient having systemic lupus erithematosus (SLE), said epitope being identical with or different in not more than 4 amino acids from an at least 20 amino acids long segment of the portion of a DNA Topoisomerase I spanning amino acids 350 to 400,
wherein the epitopes are present on separate peptides,
said one or more peptide(s) having an amino acid sequence which is at least 80% or at least 90% identical with the amino acid sequence of a peptide fragment of a wild type DNA Topoisomerase I.

6. The diagnostic kit according to claim 5 wherein said kit also comprises
c) a peptide comprising an epitope capable of binding a patient antibody of a patient having systemic sclerosis (SSC) or systemic lupus erythematosus (SLE), said epitope being identical with or different in not more than 4 amino acids from the at least 20 amino acids long segment of the portion of said DNA Topoisomerase I spanning amino acids 451 to 593,
said peptide having an amino acid sequence which is at least 80% or at least 90% identical with the amino acid sequence of a peptide fragment of a wild type DNA Topoisomerase I.

7. The diagnostic kit according to claim 6, said kit comprising
- at least one peptide selected from
a) a peptide comprising an epitope capable of binding a patient antibody of a patient having systemic lupus erithematosus (SLE), said epitope being identical with an at least 20 amino acids long segment of the portion of a DNA Topoisomerase I spanning amino acids 5 to 30,
b) a peptide comprising an epitope capable of binding a patient antibody of a patient having systemic lupus erithematosus (SLE), said epitope being identical with an at least 20 amino acids long segment of the portion of a DNA Topoisomerase I spanning amino acids 350 to 400, and
- a reference peptide comprising an epitope capable of binding a patient antibody of a patient having systemic sclerosis (SSC) or systemic lupus erythematosus (SLE), said epitope being identical with an at least 20 amino acids long segment of the portion of a DNA Topoisomerase I spanning amino acids 451 to 593; and optionally
- means for detecting binding of patient autoantibodies to a peptide epitope.

8. The diagnostic kit according to claim 7, wherein
- the peptide according to a) is a peptide fragment of said DNA Topoisomerase I spanning amino acids 5 to 30,
- the peptide according to b) is a peptide fragment of said DNA Topoisomerase I spanning amino acids 350 to 400, and
- the peptide according to c) is a peptide fragment of said DNA Topoisomerase I spanning amino acids 451 to 593.

9. A diagnostic method useful for differential diagnosis of systemic autoimmune disorders comprising the steps of
i) contacting a sample obtained from a patient with one or more peptide(s) selected from:
a) a peptide comprising an epitope capable of binding a patient antibody of a patient having diffuse cutane systemic scleroderma (dsSSC), said epitope being identical with or different in not more than 4 amino acids from an at least 20 amino acids long segment of the portion of said DNA Topoisomerase I spanning amino acids 5 to 30,
b) a peptide comprising an epitope capable of binding a patient antibody of a patient having systemic lupus erithematosus (SLE), said epitope being identical with or different in not more than 4 amino acids from an at least 20 amino acids long segment of the portion of said DNA Topoisomerase I spanning amino acids 350 to 400,
wherein the epitopes are present on separate peptides,
said one or more peptide(s) having an amino acid sequence which is at least 80% or at least 90% identical with the amino acid sequence of a peptide fragment of a wild type DNA Topoisomerase I,
wherein
- if binding of autoantibodies of said sample to epitope according to a) is detected, it is considered as indicative of diffuse cutane systemic scleroderma (dsSSC), preferably a late onset thereof, in said patient,
- if binding of autoantibodies of said sample to epitope according to b) is detected, it is considered as indicative of systemic lupus erithematosus (SLE), preferably an SLE with Raynaud's phenomenon, in said patient.

10. The diagnostic method according to claim 9), wherein the one or more isolated peptide(s) also comprise
c) a peptide comprising an epitope capable of binding a patient antibody of a patient having systemic sclerosis (SSC) or systemic lupus erythematosus (SLE), said epitope being identical with or different in not more than 4 amino acids from the at least 20 amino acids long segment of the portion of said DNA Topoisomerase I spanning amino acids 451 to 593,
said peptide having an amino acid sequence which is at least 80% or at least 90% identical with the amino acid sequence of a peptide fragment of a wild type DNA Topoisomerase I,
and wherein
- if binding of autoantibodies to epitope according to a) and c) is detected, and preferably binding of autoantibodies of said patient sample to epitope according to b) is not detected, it is considered as indicative of diffuse cutane systemic scleroderma (dsSSC) in said patient or preferably a late onset thereof,
- if binding of autoantibodies to epitope according to b) and c) is detected, and preferably binding of autoantibodies of said patient sample to epitope according to a) is not detected, it is considered as indicative of systemic lupus erithematosus (SLE) in said patient or preferably an SLE with Raynaud's phenomenon.

11. The diagnostic method of claim 9 or 10, wherein the one or more peptide(s) as defined in any of use claims 3 to 4 or kit claims 7 to 8 are used.

12. The diagnostic method of any of claims 9 to 11, the use of any of claims 1 to 4 or the diagnostic kit of any of claims 5 to 8 wherein the sample is a blood sample, preferably a serum sample.

## Patentansprüche

1. Verwendung eines oder mehrerer isolierter Peptide zur Diagnose einer systemischen Autoimmunstörung, wobei das eine oder die mehreren Peptid(e) aus den folgenden ausgewählt ist/sind:
a) einem Peptid, das ein Epitop umfasst, welches dazu befähigt ist, an einen Patientenantikörper eines Patienten zu binden, der unter diffuser kutaner systemischer Sklerodermie (dsSSC) leidet, wobei das Epitop mit einem mindestens 20 Aminosäuren langen Segment des Abschnitts einer DNA-Topoisomerase I, welcher die Aminosäuren 5 bis 30 umfasst, identisch ist oder sich von diesem um nicht mehr als 4 Aminosäuren unterscheidet,
b) einem Peptid, das ein Epitop umfasst, welches dazu befähigt ist, an einen Patientenantikörper eines Patienten zu binden, der unter systemischem Lupus erythematodes (SLE) leidet, wobei das Epitop mit einem mindestens 20 Aminosäuren langen Segment des Abschnitts einer DNA-Topoisomerase I, welcher die Aminosäuren 350 bis 400 umfasst, identisch ist oder sich von diesem um nicht mehr als 4 Aminosäuren unterscheidet,
wobei die Epitope auf gesonderten Peptiden vorliegen,
wobei das eine oder die mehreren Peptid(e) eine Aminosäuresequenz hat/haben, die zu mindestens 80% oder zu mindestens 90 % mit der Aminosäuresequenz eines Peptidfragments eines Wildtyps der DNA-Topoisomerase I identisch ist,
wobei
- der Nachweis einer Bindung von Autoantikörpern, die aus einer Probe eines Patienten stammen, an das Epitop gemäß a) als Anzeichen dafür anzusehen ist, dass bei diesem Patienten eine diffuse kutane systemische Sklerodermie (dsSSC) besteht, wobei diese vorzugsweise spät eingesetzt hat,
- der Nachweis einer Bindung von Autoantikörpern, die aus einer Probe eines Patienten stammen, an ein Epitop gemäß b) als Anzeichen dafür anzusehen ist, dass bei diesem Patienten ein systemischer Lupus erythematodes (SLE) vorliegt, wobei es sich vorzugsweise um einen SLE mit Raynaud-Syndrom handelt.

2. Verwendung nach Anspruch 1, wobei das eine oder die mehreren isolierte(n) Peptid(e) weiterhin Folgendes umfasst/umfassen
c) ein Peptid, das ein Epitop umfasst, welches dazu befähigt ist, an einen Patientenantikörper eines Patienten zu binden, der unter systemischer Sklerodermie (SSC) oder systemischem Lupus erythematodes (SLE) leidet, wobei das Epitop mit einem mindestens 20 Aminosäuren langen Segment des Abschnitts der DNA-Topoisomerase I, welcher die Aminosäuren 451 bis 593 umfasst, identisch ist oder sich von diesem um nicht mehr als 4 Aminosäuren unterscheidet,
wobei das Peptid eine Aminosäuresequenz hat, die zu mindestens 80% oder zu mindestens 90 % mit der Aminosäuresequenz eines Peptidfragments eines Wildtyps der DNA-Topoisomerase I identisch ist,
und wobei
- der Nachweis einer Bindung von Autoantikörpern, die aus einer Probe eines Patienten stammen, an das Epitop gemäß a) und c), und vorzugsweise das Ausbleiben des Nachweises der Bindung von Autoantikörpern, die aus dieser Patientenprobe stammen, an das Epitop gemäß b), als Anzeichen dafür anzusehen ist, dass bei diesem Patienten eine diffuse kutane systemische Sklerodermie (dsSSC) besteht oder dass diese vorzugsweise spät eingesetzt hat,
- der Nachweis einer Bindung von Autoantikörpern, die aus einer Probe eines Patienten stammen, an das Epitop gemäß b) und c), und vorzugsweise das Ausbleiben des Nachweises der Bindung von Autoantikörpern, die aus dieser Patientenprobe stammen, an das Epitop nach a), als Anzeichen dafür anzusehen ist, dass bei diesem Patienten ein systemischer Lupus erythematodes (SLE) oder vorzugsweise ein SLE mit Raynaud-Syndrom besteht.

3. Verwendung nach Anspruch 1 oder 2, wobei das Epitop eines beliebigen der Peptide sich von dem entsprechenden Epitop der Wildtyp-Sequenz um nicht mehr als 2 oder 1 Aminosäure unterscheidet.

4. Verwendung nach einem beliebigen der Ansprüche 1 bis 3, wobei
- es sich bei dem Epitop gemäß a) um ein mindestens 20 Aminosäuren langes Segment des Abschnitts der DNA-Topoisomerase I handelt, welcher die Aminosäuren 5 bis 30 umfasst,
- es sich bei dem Epitop gemäß b) um ein mindestens 20 Aminosäuren langes Segment des Abschnitts der DNA-Topoisomerase I handelt, welcher die Aminosäuren 350 bis 400 umfasst, und
- es sich vorzugsweise bei dem Epitop gemäß c) um ein mindestens 20 Aminosäuren langes Segment des Abschnitts der DNA-Topoisomerase I handelt, welcher die Aminosäuren 451 bis 593 umfasst.

5. Diagnose-Kit zur Verwendung bei der Diagnose einer systemischen Autoimmunstörung, wobei das Kit ein oder mehrere isolierte Peptide gemäß der Begriffsbestimmung in einem beliebigen der vorhergehenden Ansprüche umfasst,
wobei das eine oder die mehreren isolierte(n) Peptid(e) aus den folgenden ausgewählt sind:
a) einem Peptid, das ein Epitop umfasst, welches dazu befähigt ist, an einen Patientenantikörper eines Patienten zu binden, der unter diffuser kutaner systemischer Sklerodermie (dsSSC) leidet, wobei das Epitop mit einem mindestens 20 Aminosäuren langen Segment des Abschnitts einer DNA-Topoisomerase I, welcher die Aminosäuren 5 bis 30 umfasst, identisch ist oder sich von diesem um nicht mehr als 4 Aminosäuren unterscheidet,
b) einem Peptid, das ein Epitop umfasst, welches dazu befähigt ist, an einen Patientenantikörper eines Patienten zu binden, der unter systemischem Lupus erythematodes (SLE) leidet, wobei das Epitop mit einem mindestens 20 Aminosäuren langen Segment des Abschnitts einer DNA-Topoisomerase I, welcher die Aminosäuren 350 bis 400 umfasst, identisch ist oder sich von diesem um nicht mehr als 4 Aminosäuren unterscheidet,
wobei die Epitope auf gesonderten Peptiden vorliegen,
wobei das eine oder die mehreren Peptid(e) eine Aminosäuresequenz hat/haben, die zu mindestens 80% oder zu mindestens 90 % mit der Aminosäuresequenz eines Peptidfragments eines Wildtyps der DNA-Topoisomerase I identisch ist.

6. Diagnose-Kit nach Anspruch 5, wobei das Kit weiterhin Folgendes umfasst
c) ein Peptid, das ein Epitop umfasst, welches dazu befähigt ist, an einen Patientenantikörper eines Patienten zu binden, der unter systemischer Sklerodermie (SSC) oder systemischem Lupus erythematodes (SLE) leidet, wobei das Epitop mit einem mindestens 20 Aminosäuren langen Segment des Abschnitts der DNA-Topoisomerase I, welcher die Aminosäuren 451 bis 593 umfasst, identisch ist oder sich von diesem um nicht mehr als 4 Aminosäuren unterscheidet, wobei das Peptid eine Aminosäuresequenz hat, die zu mindestens 80% oder zu mindestens 90 % mit der Aminosäuresequenz eines Peptidfragments eines Wildtyps der DNA-Topoisomerase I identisch ist.

7. Diagnose-Kit nach Anspruch 6, wobei das Kit weiterhin Folgendes umfasst
- mindestens ein Peptid, das aus den folgenden ausgewählt ist
a) einem Peptid, das ein Epitop umfasst, welches dazu befähigt ist, an einen Patientenantikörper eines Patienten zu binden, der unter systemischem Lupus erythematodes (SLE) leidet, wobei das Epitop mit einem mindestens 20 Aminosäuren langen Segment des Abschnitts einer DNA-Topoisomerase I, welcher die Aminosäuren 5 bis 30 umfasst, identisch ist,
b) einem Peptid, das ein Epitop umfasst, welches dazu befähigt ist, an einen Patientenantikörper eines Patienten zu binden, der unter systemischem Lupus erythematodes (SLE) leidet, wobei das Epitop mit einem mindestens 20 Aminosäuren langen Segment des Abschnitts einer DNA-Topoisomerase I, welcher die Aminosäuren 350 bis 400 umfasst, identisch ist, und
- ein Referenz-Peptid, das ein Epitop umfasst, welches dazu befähigt ist, an einen Patientenantikörper eines Patienten zu binden, der unter systemischer Sklerodermie (SSC) oder systemischem Lupus erythematodes (SLE) leidet, wobei das Epitop mit einem mindestens 20 Aminosäuren langen Segment des Abschnitts der DNA-Topoisomerase I, welcher die Aminosäuren 451 bis 593 umfasst, identisch ist; sowie möglicherweise
- Mittel zum Nachweis der Bindung von Patienten-Autoantikörpern an ein Peptidepitop.

8. Diagnose-Kit nach Anspruch 7, wobei
- es sich bei dem Peptid gemäß a) um ein Peptidfragment der DNA-Topoisomerase I handelt, welches die Aminosäuren 5 bis 30 umfasst,
- es sich bei dem Peptid gemäß b) um ein Peptidfragment der DNA-Topoisomerase I handelt, welches die Aminosäuren 350 bis 400 umfasst, und
- es sich bei dem Peptid gemäß c) um ein Peptidfragment der DNA-Topoisomerase I handelt, welches die Aminosäuren 451 bis 593 umfasst.

9. Diagnoseverfahren, das zur Differentialdiagnose von systemischen Autoimmunstörungen verwendet werden kann und die folgenden Schritte umfasst
i) Inkontaktbringen einer Probe, die von einem Patienten stammt, mit einem oder mehreren Peptid(en), das/die aus den folgenden ausgewählt ist/sind:
a) einem Peptid, das ein Epitop umfasst, welches dazu befähigt ist, an einen Patientenantikörper eines Patienten zu binden, der unter diffuser kutaner systemischer Sklerodermie (dsSSC) leidet, wobei das Epitop mit einem mindestens 20 Aminosäuren langen Segment des Abschnitts einer DNA-Topoisomerase I, welcher die Aminosäuren 5 bis 30 umfasst, identisch ist oder sich von diesem um nicht mehr als 4 Aminosäuren unterscheidet,
b) einem Peptid, das ein Epitop umfasst, welches dazu befähigt ist, an einen Patientenantikörper eines Patienten zu binden, der unter systemischem Lupus erythematodes (SLE) leidet, wobei das Epitop mit einem mindestens 20 Aminosäuren langen Segment des Abschnitts einer DNA-Topoisomerase I, welcher die Aminosäuren 350 bis 400 umfasst, identisch ist oder sich von diesem um nicht mehr als 4 Aminosäuren unterscheidet,
wobei die Epitope auf gesonderten Peptiden vorliegen,
wobei das eine oder die mehreren Peptid(e) eine Aminosäuresequenz hat/haben, die zu mindestens 80% oder zu mindestens 90 % mit der Aminosäuresequenz eines Peptidfragments eines Wildtyps der DNA-Topoisomerase I identisch ist,
wobei
- der Nachweis einer Bindung von Autoantikörpern, die aus dieser Probe stammen, an das Epitop gemäß a) als Anzeichen dafür anzusehen ist, dass bei diesem Patienten eine diffuse kutane systemische Sklerodermie (dsSSC) besteht, wobei diese vorzugsweise spät eingesetzt hat,
- der Nachweis einer Bindung von Autoantikörpern, die aus dieser Probe stammen, an ein Epitop gemäß b) als Anzeichen dafür anzusehen ist, dass bei diesem Patienten ein systemischer Lupus erythematodes (SLE) vorliegt, wobei es sich vorzugsweise um einen SLE mit Raynaud-Syndrom handelt.

10. Diagnoseverfahren nach Anspruch 9, wobei das eine oder die mehreren isolierte(n) Peptid(e) weiterhin Folgendes umfasst/umfassen
c) ein Peptid, das ein Epitop umfasst, welches dazu befähigt ist, an einen Patientenantikörper eines Patienten zu binden, der unter systemischer Sklerodermie (SSC) oder systemischem Lupus erythematodes (SLE) leidet, wobei das Epitop mit einem mindestens 20 Aminosäuren langen Segment des Abschnitts der DNA-Topoisomerase I, welcher die Aminosäuren 451 bis 593 umfasst, identisch ist oder sich von diesem um nicht mehr als 4 Aminosäuren unterscheidet,
wobei das Peptid eine Aminosäuresequenz hat, die zu mindestens 80% oder zu mindestens 90 % mit der Aminosäuresequenz eines Peptidfragments eines Wildtyps der DNA-Topoisomerase I identisch ist,
und wobei
- der Nachweis einer Bindung von Autoantikörpern an das Epitop gemäß a) und c), und vorzugsweise das Ausbleiben des Nachweises der Bindung von Autoantikörpern, die aus dieser Patientenprobe stammen, an das Epitop gemäß b), als Anzeichen dafür anzusehen ist, dass bei diesem Patienten eine diffuse kutane systemische Sklerodermie (dsSSC) besteht oder dass diese vorzugsweise spät eingesetzt hat,
- der Nachweis einer Bindung von Autoantikörpern an das Epitop gemäß b) und c), und vorzugsweise das Ausbleiben des Nachweises der Bindung von Autoantikörpern, die aus dieser Patientenprobe stammen, an das Epitop nach a), als Anzeichen dafür anzusehen ist, dass bei diesem Patienten ein systemischer Lupus erythematodes (SLE) oder vorzugsweise ein SLE mit Raynaud-Syndrom besteht.

11. Diagnoseverfahren nach Anspruch 9 oder 10, wobei das eine oder die mehreren Peptid(e) gemäß der Begriffsbestimmung in einem beliebigen der Verwendungsansprüche 3 bis 4 oder der Kit-Ansprüche 7 bis 8 zur Anwendung kommt/kommen.

12. Diagnoseverfahren nach einem beliebigen der Ansprüche 9 bis 11, Verwendung nach einem beliebigen der Ansprüche 1 bis 4 oder Diagnose-Kit nach einem beliebigen der Ansprüche 5 bis 8, wobei es sich bei der Probe um eine Blutprobe, vorzugsweise um eine Serumprobe, handelt.

## Revendications

1. Utilisation d'un ou de plusieurs peptide(s) isolé(s) pour le diagnostic d'un trouble auto-immun systémique, ledit(lesdits) un ou plusieurs peptide(s) étant choisi(s) parmi :
a) un peptide comprenant un épitope capable de se lier à un anticorps de patient d'un patient présentant une sclérose systémique cutanée diffuse (ScScd), ledit épitope étant identique à ou différent de pas plus de 4 acides aminés d'un segment long d'au moins 20 acides aminés de la portion d'une ADN topoisomérase I s'étendant des acides aminés 5 à 30,
b) un peptide comprenant un épitope capable de se lier à un anticorps de patient d'un patient présentant un lupus érythémateux disséminé (LED), ledit épitope étant identique à ou différent de pas plus de 4 acides aminés d'un segment long d'au moins 20 acides aminés de la portion d'une ADN topoisomérase I s'étendant des acides aminés 350 à 400,
dans lequel les épitopes sont présents sur des peptides distincts,
ledit(lesdits) un ou plusieurs peptide(s) ayant une séquence d'acides aminés qui est identique à au moins 80 % ou au moins 90 % à la séquence d'acides aminés d'un fragment peptidique d'une ADN topoisomérase I de type sauvage, dans laquelle
- si une liaison des auto-anticorps d'un échantillon provenant d'un patient à un épitope selon a) est détectée, elle est considérée comme indicatrice d'une sclérodermie systémique cutanée diffuse (ScScd), de préférence une apparition tardive de celle-ci, chez ledit patient,
- si une liaison des auto-anticorps d'un échantillon provenant d'un patient à un épitope selon b) est détectée, elle est considérée comme indicatrice d'un lupus érythémateux disséminé (LED), de préférence un LED avec le phénomène de Raynaud, chez ledit patient.

2. Utilisation selon la revendication 1, dans laquelle le(s) un ou plusieurs peptide(s) isolé(s) comprend(comprennent) en outre
c) un peptide comprenant un épitope capable de se lier à un anticorps de patient d'un patient présentant une sclérodermie systémique (ScS) ou un lupus érythémateux disséminé (LED), ledit épitope étant identique à ou différent de pas plus de 4 acides aminés du segment long d'au moins 20 acides aminés de la portion de ladite ADN topoisomérase I s'étendant des acides aminés 451 à 593,
ledit peptide ayant une séquence d'acides aminés qui est identique à au moins 80 % ou au moins 90 % à la séquence d'acides aminés d'un fragment peptidique d'une ADN topoisomérase I de type sauvage,
et dans laquelle
- si une liaison des auto-anticorps d'un échantillon provenant d'un patient à un épitope selon a) et c) est détectée, et de préférence une liaison des auto-anticorps dudit échantillon de patient à un épitope selon b) n'est pas détectée, elle est considérée comme indicatrice d'une sclérodermie systémique cutanée diffuse (ScScd) chez ledit patient ou de préférence une apparition tardive de celle-ci,
- si une liaison des auto-anticorps d'un échantillon provenant d'un patient à un épitope selon b) et c) est détectée, et de préférence une liaison des auto-anticorps dudit échantillon de patient à un épitope selon a) n'est pas détectée, elle est considérée comme indicatrice d'un lupus érythémateux disséminé (LED) chez ledit patient ou de préférence un LED avec le phénomène de Raynaud.

3. Utilisation selon la revendication 1 ou 2, dans laquelle l'épitope de l'un quelconque desdits peptides est différent de pas plus de 2 ou 1 acide(s) aminé(s) de l'épitope respectif de la séquence de type sauvage.

4. Utilisation selon l'une quelconque des revendications 1 à 3, dans laquelle
- un épitope selon a) est un segment long d'au moins 20 acides aminés de la portion de ladite ADN topoisomérase I s'étendant des acides aminés 5 à 30,
- un épitope selon b) est un segment long d'au moins 20 acides aminés de la portion de ladite ADN topoisomérase I s'étendant des acides aminés 350 à 400, et de préférence
- un épitope selon c) est un segment long d'au moins 20 acides aminés de la portion de ladite ADN topoisomérase I s'étendant des acides aminés 451 à 593.

5. Trousse de diagnostic destinée à une utilisation dans le diagnostic d'un trouble auto-immun systémique, ladite trousse comprenant un ou plusieurs peptides isolés tels que définis selon l'une quelconque des revendications précédentes,
ledit(lesdits) un ou plusieurs peptide(s) isolé(s) étant choisi(s) parmi :
a) un peptide comprenant un épitope capable de se lier à un anticorps de patient d'un patient présentant une sclérodermie systémique cutanée diffuse (ScScd), ledit épitope étant identique à ou différent de pas plus de 4 acides aminés d'un segment long d'au moins 20 acides aminés de la portion d'une ADN topoisomérase I s'étendant des acides aminés 5 à 30,
b) un peptide comprenant un épitope capable de se lier à un anticorps de patient d'un patient présentant un lupus érythémateux disséminé (LED), ledit épitope étant identique à ou différent de pas plus de 4 acides aminés d'un segment long d'au moins 20 acides aminés de la portion d'une ADN topoisomérase I s'étendant des acides aminés 350 à 400,
dans laquelle les épitopes sont présents sur des peptides distincts,
ledit(lesdits) un ou plusieurs peptide(s) ayant une séquence d'acides aminés qui est identique à au moins 80 % ou au moins 90 % à la séquence d'acides aminés d'un fragment peptidique d'une ADN topoisomérase I de type sauvage.

6. Trousse de diagnostic selon la revendication 5, dans laquelle ladite trousse comprend en outre
c) un peptide comprenant un épitope capable de se lier à un anticorps de patient d'un patient présentant une sclérodermie systémique (ScS) ou un lupus érythémateux disséminé (LED), ledit épitope étant identique à ou différent de pas plus de 4 acides aminés du segment long d'au moins 20 acides aminés de la portion de ladite ADN topoisomérase I s'étendant des acides aminés 451 à 593,
ledit peptide ayant une séquence d'acides aminés qui est identique à au moins 80 % ou au moins 90 % à la séquence d'acides aminés d'un fragment peptidique d'une ADN topoisomérase I de type sauvage.

7. Trousse de diagnostic selon la revendication 6, ladite trousse comprenant
- au moins un peptide choisi parmi :
a) un peptide comprenant un épitope capable de se lier à un anticorps de patient d'un patient présentant un lupus érythémateux disséminé (LED), ledit épitope étant identique à un segment long d'au moins 20 acides aminés de la portion d'une ADN topoisomérase I s'étendant des acides aminés 5 à 30,
b) un peptide comprenant un épitope capable de se lier à un anticorps de patient d'un patient présentant un lupus érythémateux disséminé (LED), ledit épitope étant identique à un segment long d'au moins 20 acides aminés de la portion d'une ADN topoisomérase I s'étendant des acides aminés 350 à 400, et
- un peptide de référence, comprenant un épitope capable de se lier à un anticorps de patient d'un patient présentant une sclérodermie systémique (ScS) ou un lupus érythémateux disséminé (LED), ledit épitope étant identique à un segment long d'au moins 20 acides aminés de la portion d'une ADN topoisomérase I s'étendant des acides aminés 451 à 593 ; et éventuellement
- un moyen de détection de la liaison des auto-anticorps du patient à un épitope peptidique.

8. Trousse de diagnostic selon la revendication 7, dans laquelle
- le peptide selon a) est un fragment peptidique de ladite ADN topoisomérase I s'étendant des acides aminés 5 à 30,
- le peptide selon b) est un fragment peptidique de ladite ADN topoisomérase I s'étendant des acides aminés 350 à 400, et
- le peptide selon c) est un fragment peptidique de ladite ADN topoisomérase I s'étendant des acides aminés 451 à 593.

9. Procédé de diagnostic utile pour le diagnostic différentiel des troubles auto-immuns systémiques, comprenant les étapes consistant à
i) mettre en contact un échantillon prélevé chez un patient avec un ou plusieurs peptide(s) choisi(s) parmi :
a) un peptide comprenant un épitope capable de se lier à un anticorps de patient d'un patient présentant une sclérodermie systémique cutanée diffuse (ScScd), ledit épitope étant identique à ou différent de pas plus de 4 acides aminés d'un segment long d'au moins 20 acides aminés de la portion de ladite ADN topoisomérase I s'étendant des acides aminés 5 à 30,
b) un peptide comprenant un épitope capable de se lier à un anticorps de patient d'un patient présentant un lupus érythémateux disséminé (LED), ledit épitope étant identique à ou différent de pas plus de 4 acides aminés d'un segment long d'au moins 20 acides aminés de la portion de ladite ADN topoisomérase I s'étendant des acides aminés 350 à 400,
dans lequel les épitopes sont présents sur des peptides distincts,
ledit(lesdits) un ou plusieurs peptide(s) ayant une séquence d'acides aminés qui est identique à au moins 80 % ou au moins 90 % à la séquence d'acides aminés d'un fragment peptidique d'une ADN topoisomérase I de type sauvage, dans lequel
- si une liaison des auto-anticorps dudit échantillon à un épitope selon a) est détectée, elle est considérée comme indicatrice d'une sclérodermie systémique cutanée diffuse (ScScd), de préférence une apparition tardive de celle-ci, chez ledit patient,
- si une liaison des auto-anticorps dudit échantillon à un épitope selon b) est détectée, elle est considérée comme indicatrice d'un lupus érythémateux disséminé (LED), de préférence un LED avec le phénomène de Raynaud, chez ledit patient.

10. Procédé de diagnostic selon la revendication 9), dans lequel le(s) un ou plusieurs peptide(s) isolé(s) comprend(comprennent) en outre
c) un peptide comprenant un épitope capable de se lier à un anticorps de patient d'un patient présentant une sclérodermie systémique (ScS) ou un lupus érythémateux disséminé (LED), ledit épitope étant identique à ou différent de pas plus de 4 acides aminés d'un segment long d'au moins 20 acides aminés de la portion de ladite ADN topoisomérase I s'étendant des acides aminés 451 à 593,
ledit peptide ayant une séquence d'acides aminés qui est identique à au moins 80 % ou au moins 90 % à la séquence d'acides aminés d'un fragment peptidique d'une ADN topoisomérase I de type sauvage,
et dans lequel
- si une liaison des auto-anticorps à un épitope selon a) et c) est détectée, et de préférence une liaison des auto-anticorps dudit échantillon de patient à un épitope selon b) n'est pas détectée, elle est considérée comme indicatrice d'une sclérodermie systémique cutanée diffuse (ScScd) chez ledit patient ou de préférence une apparition tardive de celle-ci,
- si une liaison des auto-anticorps à un épitope selon b) et c) est détectée, et de préférence une liaison des auto-anticorps dudit échantillon de patient à un épitope selon a) n'est pas détectée, elle est considérée comme indicatrice d'un lupus érythémateux disséminé (LED) chez ledit patient ou de préférence un LED avec le phénomène de Raynaud.

11. Procédé de diagnostic selon la revendication 9 ou 10, dans lequel le(s) un ou plusieurs peptide(s) tel(s) que défini(s) selon l'une quelconque des revendications d'utilisation 3 à 4 ou des revendications de trousse 7 à 8 sont utilisés.

12. Procédé de diagnostic selon l'une quelconque des revendications 9 à 11, l'utilisation selon l'une quelconque des revendications 1 à 4 ou la trousse de diagnostic selon l'une quelconque des revendications 5 à 8, dans lequel l'échantillon est un échantillon de sang, de préférence un échantillon de sérum.
